# EUROPEAN PATENT SPECIFICATION

(11) **EP 4 221 591 B1**
(45) Date of publication and mention of the grant of the patent: **28.10.2026**
(21) Application number: 21876082.5
(22) Date of filing: 28.09.2021
(51) Int. Cl.: A61M 25/01, A61B 5/20, A61M 25/10, A61B 1/307, A61M 25/00, A61M 25/04

(54) **A DIAGNOSTIC KIT AND METHOD THEREFORE**
DIAGNOSEKIT UND VERFAHREN DAFÜR
KIT DE DIAGNOSTIC ET PROCÉDÉ ASSOCIÉ

(30) Priority: 01.10.2020 SE 2051143
(43) Date of publication of application: 09.08.2023
(73) Proprietor: Prostalund AB, 223 63 Lund (SE)
(72) Inventor: SCHELIN, Sonny, 395 95 ROCKNEBY (SE); ISRAELSSON, Anette, 226 47 LUND (SE); WENNERHOLM, Johan, 234 42 LOMMA (SE)
(74) Representative: AWA Sweden AB
(86) International application number: PCT/SE2021/050946
(87) International publication number: WO 2022/071850

(56) References cited:
- WO-A1-2019/051490
- WO-A2-02/17990
- US-A1- 2006 111 691
- US-A1- 2006 111 691
- US-A1- 2013 277 248
- US-B1- 10 737 057

## Description

### Technical Field

The present disclosure relates to a diagnostic kit and method intended for diagnosis of a patient before a potential prostate surgery or treatment, such as e.g. an invasive prostate therapy.

### Background

US 2006/0111691 A1 discloses a partial-length catheter, or an extendable tube or sleeve member of the catheter, which is selectively movable within the prostatic urethra to open a urine drainage passageway through and obstructed portion of the prostatic urethra or to open the external urinary sphincter muscle and thereby discharge urine from the bladder. A control element is manipulated at a position exterior of the urinary canal to selectively move the catheter or the extendable tube or sleeve member, thereby selectively controlling urine discharge. The catheter may also be used to diagnose urinary retention problems caused by a weak bladder or a prostatic obstruction.

### Summary

The present disclosure refers to a diagnostic kit comprising
- an indwelling catheter comprising two connectable and separable main units being
- an introduction stent unit with a top and bottom portion, said introduction stent unit also comprising a bladder fluid opening, wherein said introduction stent unit has a length possible to arrange with the top inside of the bladder of a patient and with the bottom portion arranged inside of the urethra before the external urinary sphincter muscle of the patient when seen from the bladder and outwards;
- a catheter filling unit with an open bottom end and a top portion, said catheter filling unit being detachable form the indwelling catheter;
   said indwelling catheter also comprising
- a balloon stopper unit in connection with and thus possible to fill via a balloon filling tube and out from a balloon fluid inlet opening arranged on the introduction stent unit, wherein the filling of the balloon stopper unit with a sterile fluid, preferably sterile water, is intended when the balloon stopper unit is arranged inside of the bladder of the patient to provide for a stopper function of the balloon stopper unit against the bladder neck of the patient; and
- a pull thread fixedly arranged on the introduction stent unit to enable pulling of the introduction stent unit when the catheter filling unit is detached from the indwelling catheter;
   said diagnostic kit also comprising
- a set volume of a saline solution (or corresponding solution) contained in a syringe connectable to the open bottom end of the catheter filling unit; and
- a drainage and diagnosis container with volume markings.

Moreover, the present disclosure also refers to a method for performing a diagnostic test before a potential prostate surgery/treatment, said method comprising:
- arranging an indwelling catheter inside of the urethra and up into the bladder of a patient,
   said indwelling catheter comprising two connectable and separable main units being
   - an introduction stent unit with a top and bottom portion, said introduction stent unit also comprising a bladder fluid opening, wherein said introduction stent unit has a length possible to arrange with the top inside of the bladder of a patient and with the bottom portion arranged inside of the urethra before the external urinary sphincter muscle of the patient when seen from the bladder and outwards;
   - a catheter filling unit with an open bottom end and a top portion, said catheter filling unit being detachable form the indwelling catheter;
      said indwelling catheter also comprising
   - a balloon stopper unit in connection with and thus possible to fill via a balloon filling tube and out from a balloon fluid inlet opening arranged on the introduction stent unit, wherein the filling of the balloon stopper unit is intended when the balloon stopper unit is arranged inside of the bladder of the patient to provide for a stopper function of the balloon stopper unit against the bladder neck of the patient; and
   - a pull thread fixedly arranged on the introduction stent unit to enable pulling of the introduction stent unit when the catheter filling unit is detached from the indwelling catheter;
   said method also comprising
- filling the balloon stopper unit with a sterile fluid, preferably sterile water, to fixedly arrange the top of the introduction stent unit inside of the bladder of the patient;
- flowing a volume of saline solution (or corresponding solution) contained in a syringe being connected to the open bottom end of the catheter filling unit into the bladder of the patient, preferably until the patient is in need or urinating;
- noting the volume filled into the bladder;
- detaching the catheter filling unit from the indwelling catheter;
- instructing the patient to perform certain movements during a certain time frame;
- organizing for patient-driven urination from the patient into a drainage and diagnosis container with volume markings;
- noting the urinated volume from the patient; and finally
- performing a diagnosis based on the calculation of urinated volume in relation to the volume of saline solution (or corresponding solution) flown into the bladder of the patient.

### Specific embodiments of the disclosure

Some specific embodiments of the present disclosure are provided below and in the attached claims. This is both valid for the diagnostic kit and for the method and use according to the present disclosure.

Moreover, below there is presented one alternative of an indwelling catheter for a diagnostic kit according to one specific embodiment of the present disclosure.

This alternative comprises an indwelling catheter 1 comprising:
an introduction stent unit 2 with a top 2a and bottom portion 2b, and a bladder fluid opening 20;
a catheter filling unit 3 with an open bottom end 3a and a top portion 3b;
a balloon stopper unit 4 in connection with a balloon filling tube 5;
a balloon fluid inlet opening 21 arranged on the introduction stent unit 2;
the balloon filling tube (5) has a portion (50) with a spiral shape along the balloon filling tube (5) at a position outside and a distance from the bottom portion (2b) of the introduction stent unit (2), to enable anchoring inside of the urethra and outside of the external urinary sphincter muscle of the patient when seen from the bladder and outwards;
a receptacle 51 situated on the end of the balloon filling tube 5 before a valve arrangement 52;
   and
a pull thread 6 fixedly arranged on the introduction stent unit 2.

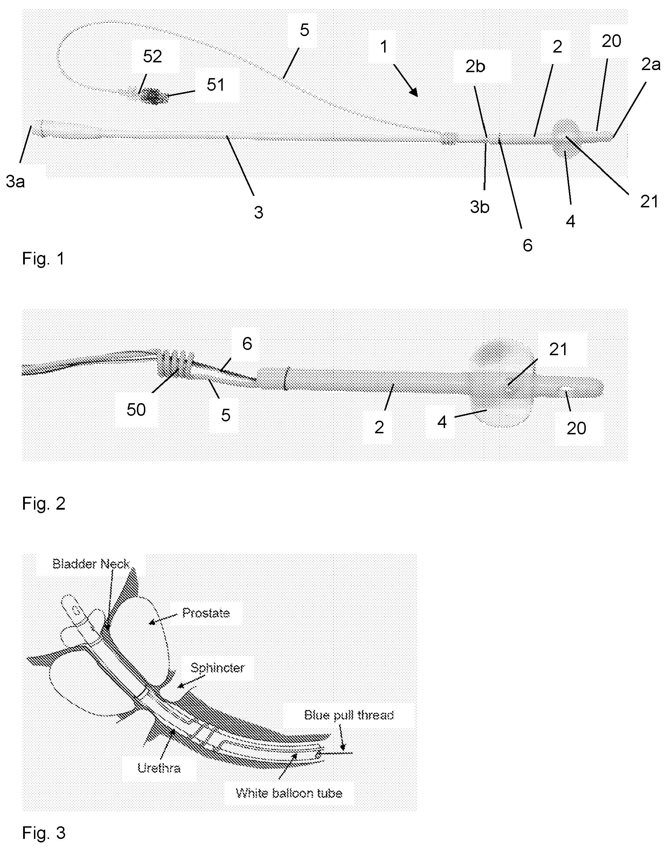

One embodiment thereof is presented in figs. 1-2.

In relation to the above it should be noted that the diagnostic kit according to the present disclosure also comprises a set volume of a saline solution (or corresponding solution) contained in a syringe connectable to the open bottom end of the catheter filling unit; and a drainage and diagnosis container with volume markings, which both are not shown above. For these two units, different shapes and sizes etc. may be used according to the present disclosure.

Moreover, according to one specific embodiment, the balloon filling tube has a portion with a spiral shape along the balloon filling tube at a position outside and a distance from the bottom portion of the introduction stent unit, to enable anchoring inside of the urethra and outside of the external urinary sphincter muscle of the patient when seen from the bladder and outwards. One alternative therefore is shown above. This anchoring ensures to hold the stent unit in position so this does not enter up into the bladder too much. In the same way, the balloon unit ensures that the stent unit is not pushed out from the position in the bladder.

Furthermore, and in reference to the figures above, according to one specific embodiment, the diagnostic kit also comprises a balloon filling syringe connectable to the balloon filling tube, preferably connectable to a receptacle situated on the end of the balloon filling tube, more preferably connectable to a receptacle situated on the end of the balloon filling tube before a valve arrangement, preferably wherein said balloon filling syringe comprises sterile fluid, suitably sterile water, intended to fill the balloon stopper unit.

Moreover, according to the present disclosure the optimal length of the introduction stent unit is in the range of 30 - 70 mm, from the top to the bottom portion of the introduction stent unit.

As should be understood from above, the present disclosure is also directed to a diagnostic method. According to one specific embodiment, the diagnosis is directed to evaluating if a patient is a suitable candidate for prostate surgery or treatment based on the calculation of urinated volume / volume of saline solution (or corresponding solution) flown into the bladder.

Furthermore, according to yet another embodiment, the diagnosis is directed to evaluating if a patient is a suitable candidate for prostate surgery or treatment based on the urinated volume during a set time range, preferably a time range of 30 seconds or less, more preferably a time range of 15 seconds.

Moreover, according to yet another embodiment of the present disclosure, the diagnosis also include at least one of the following:
- evaluation with reference to if the patient is able to urinate on request;
- evaluation with reference to if the patient is able to avoid leakage during movement.

In relation to the diagnostic method according to the present disclosure, the following criteria are such showing a strong result indicating that the patient is suitable for a prostate surgery/treatment:
- the patient is able to urinate on request (this also implies the ability of stopping the urination on a request and starting the same again thereafter);
- the patient is able to avoid leakage during movement;
- the patient is able to urinate at least 1 dl during a time frame of less than 15 seconds; and
- the patient is able to urinate at least 50% of the saline solution flown into the bladder.

The diagnostic method according to the present disclosure also provides insight on the patient's coordination ability of the bladder and the outer sphincter muscle, i.e. the patient's ability to avoid urination when the bladder is full and using the outer sphincter muscle to ensure this.

Moreover, the present disclosure is also directed to use of a diagnostic kit according to the present disclosure, for performing a diagnosis directed to evaluating if a patient is a suitable candidate for prostate surgery/treatment or not based on the calculation of urinated volume / volume of saline solution (or corresponding solution) flown into the bladder after performing filling a volume of saline solution (or corresponding solution) into the bladder of the patient and measuring the volume of urine from patient-driven urination after detachment of the catheter filling unit from the indwelling catheter.

Furthermore, the present disclosure also refers to use of a diagnostic kit according to the present disclosure, for performing a method according to the present disclosure, both as described above and in the claims.

Below there is presented more background and figures in relation to the diagnostic kit with an indwelling catheter according to the present disclosure.

Furthermore, there is also presented more information in relation to the diagnostic method according to the present disclosure.

Firstly, stent during insertion is presented in fig. 3 Insertion may be performed in accordance with the following
- Apply anesthetic gel
- Insert
- Fill balloon, tie knots on balloon tube, cut off the valve
- Empty the bladder
- Fill the bladder to prepare for voiding tests
- Separate rear part

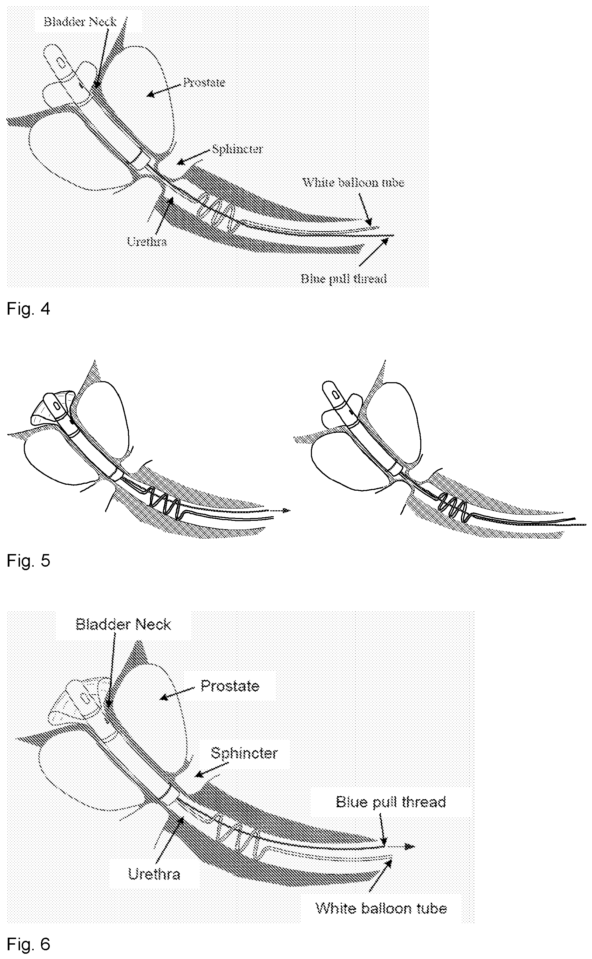

Secondly, self-controlled voiding is presented below in figs. 4-5
- Open and close sphincter - physician (see fig. 5)
- Open and close sphincter - patient (see fig. 5)

Moreover, self-catheterization is presented in fig. 6

Some functionality of the present disclosure
- Before separation the device functions as an ordinary "Foley" Catheter. Pull thread locks front and rear section together.
- Relieves obstruction in the prostatic urethra.
- Balloon and anchoring device holds stent in place,
- Migrated stent, that may cause retention, can be repositioned by the patient himself.
- Micturation can be patient-controlled using the "pull thread".

As may be understood from above, the diagnostic kit according to the present disclosure has multi-functionality. The diagnostic kit provides for the following capabilities:
- catheter function;
- stent function;
- diagnostic kit test (urodynamic test) as described above;
- self-catheterization;

With reference to the diagnostic test capability, this may provide for:
- Simulates removal of obstruction;
- Provides an answer to if the patient benefit from an invasive therapy, with answers to questions such as: Is he incontinent? / Does he have normal bladder function?

## Claims

1. A diagnostic kit comprising
- an indwelling catheter (1) comprising two connectable and separable main units being
- an introduction stent unit (2) with a top (2a) and bottom portion (2b), said introduction stent unit (2) also comprising a bladder fluid opening (20), wherein said introduction stent unit (2) has a length possible to arrange with the top (2a) inside of the bladder of a patient and with the bottom portion (2b) arranged inside of the urethra before the external urinary sphincter muscle of the patient when seen from the bladder and outwards, wherein the introduction stent unit (2) from the top (2a) to the bottom portion (2b) has a total length in the range of 30 - 70 mm;
- a catheter filling unit (3) with an open bottom end (3a) and a top portion (3b), said catheter filling unit (3) being detachable form the indwelling catheter (1);
said indwelling catheter (1) also comprising
- a balloon stopper unit (4) in connection with and thus possible to fill via a balloon filling tube (5) and out from a balloon fluid inlet opening (21) arranged on the introduction stent unit (2), wherein the filling of the balloon stopper unit (4) with a sterile fluid, preferebly sterile water, is intended when the balloon stopper unit (4) is arranged inside of the bladder of the patient to provide for a stopper function of the balloon stopper unit (4) against the bladder neck of the patient; and
- a pull thread (6) fixedly arranged on the introduction stent unit (2) to enable pulling of the introduction stent unit (2) when the catheter filling unit (3) is detached from the indwelling catheter (1);
said diagnostic kit also comprising
- a set volume of saline solution, or a corresponding solution, contained in a syringe connectable to the open bottom end (3a) of the catheter filling unit (3); and
- a drainage and diagnosis container with volume markings;
said diagnostic kit also comprising a balloon filling syringe connectable to the balloon filling tube (5), preferably connectable to a receptacle (51) situated on the end of the balloon filling tube (5) before a valve arrangement (52),
wherein said balloon filling syringe comprises sterile fluid intended to fill the balloon stopper unit (4).

2. The diagnostic kit according to claim 1, wherein the balloon filling tube (5) has a portion (50) with a spiral shape along the balloon filling tube (5) at a position outside and a distance from the bottom portion (2b) of the introduction stent unit (2), to enable anchoring inside of the urethra and outside of the external urinary sphincter muscle of the patient when seen from the bladder and outwards.

3. The diagnostic kit according to claim 1 or 2, wherein the sterile fluid is sterile water.

4. Use of a diagnostic kit according to any of claims 1-3, for performing a diagnosis directed to evaluating if a patient is a suitable candidate for prostate surgery/treatment or not based on the calculation of urinated volume / volume of saline solution, or corresponding solution, flown into the bladder after performing filling a volume of saline solution, or corresponding solution, into the bladder of the patient and measuring the volume of urine from patient-driven urination after detachment of the catheter filling unit (3) from the indwelling catheter (1).

## Patentansprüche

1. Diagnosekit, das Folgendes umfasst:
- einen Verweilkatheter (1) mit zwei verbindbaren und trennbaren Haupteinheiten, die Folgendes sind
- eine Stenteinführeinheit (2) mit einem oberen (2a) und einem unteren Abschnitt (2b), wobei die Stenteinführeinheit (2) auch eine Blasenfluidöffnung (20) umfasst, wobei die Stenteinführeinheit (2) eine Länge aufweist, die so angeordnet werden kann, dass sich der obere Abschnitt (2a) innerhalb der Blase eines Patienten befindet und dass der untere Abschnitt (2b) innerhalb der Harnröhre vor dem äußeren Harnröhrenschließmuskel des Patienten angeordnet ist, wenn von der Blase aus und von außen gesehen, wobei die Stenteinführeinheit (2) vom oberen (2a) zum unteren Abschnitt (2b) eine Gesamtlänge im Bereich von 30 - 70 mm aufweist;
- eine Katheterfülleinheit (3) mit einem offenen unteren Ende (3a) und einem oberen Abschnitt (3b), wobei die Katheterfülleinheit (3) vom Verweilkatheter (1) lösbar ist;
wobei der Verweilkatheter (1) auch Folgendes umfasst
- eine Ballonanschlageinheit (4), in Verbindung mit einem Ballonfüllrohr (5) und somit über dieses füllbar, und außerhalb einer an der Stenteinführeinheit (2) angeordneten Ballonfluideinlassöffnung (21), wobei das Befüllen der Ballonanschlageinheit (4) mit einem sterilen Fluid, vorzugsweise sterilem Wasser, beabsichtigt ist, wenn die Ballonanschlageinheit (4) innerhalb der Blase des Patienten angeordnet ist, um eine Anschlagfunktion der Ballonanschlageinheit (4) am Blasenhals des Patienten bereitzustellen; und
- einen Zugfaden (6), der fest an der Stenteinführeinheit (2) angeordnet ist, um ein Ziehen der Stenteinführeinheit (2) zu ermöglichen, wenn die Katheterfülleinheit (3) vom Verweilkatheter (1) gelöst wird;
wobei das Diagnosekit auch Folgendes umfasst
- ein festgelegtes Volumen an Kochsalzlösung oder einer entsprechenden Lösung, die in einer Spritze enthalten ist, die mit dem offenen unteren Ende (3a) der Katheterfülleinheit (3) verbindbar ist; und
- einen Ablass- und Diagnosebehälter mit Volumenmarkierungen;
wobei das Diagnosekit ferner eine Ballonfüllspritze umfasst, die mit dem Ballonfüllrohr (5) verbindbar ist, vorzugsweise mit einer am Ende des Ballonfüllrohrs (5) befindlichen Aufnahme (51) vor einer Ventilanordnung (52) verbindbar ist, wobei die Ballonfüllspritze steriles Fluid zum Füllen der Ballonanschlageinheit (4) umfasst.

2. Diagnosekit nach Anspruch 1, wobei das Ballonfüllrohr (5) einen Abschnitt (50) mit einer spiralförmigen Form entlang des Ballonfüllrohrs (5) an einer Position außerhalb und in einem Abstand von dem unteren Abschnitt (2b) der Stenteinführeinheit (2) aufweist, um eine Verankerung innerhalb der Harnröhre und außerhalb des äußeren Harnröhrenschließmuskels des Patienten zu ermöglichen, wenn von der Blase aus und nach außen gesehen.

3. Diagnosekit nach Anspruch 1 oder 2, wobei das sterile Fluid steriles Wasser ist.

4. Verwendung eines Diagnosekits nach einem der Ansprüche 1-3 zum Durchführen einer Diagnose, die darauf gerichtet ist, zu bewerten, ob ein Patient ein geeigneter Kandidat für eine Prostataoperation/-behandlung ist oder nicht, basierend auf der Berechnung von uriniertem Volumen/ Volumen von Salzlösung oder einer entsprechenden Lösung, die nach dem Durchführen des Füllens eines Volumens von Salzlösung oder einer entsprechenden Lösung in die Blase geflossen ist, in die Blase des Patienten und Messen des Urinvolumens aus dem patientengetriebenen Urinieren nach Ablösen der Katheterfülleinheit (3) vom Verweilkatheter (1).

## Revendications

1. Nécessaire de diagnostic, comprenant
- un cathéter à demeure (1) comprenant deux unités principales raccordables et séparables, qui sont
- une unité d'endoprothèse d'introduction (2) avec une partie supérieure (2a) et une portion inférieure (2b), ladite unité d'endoprothèse d'introduction (2) comprenant également une ouverture de fluide de vessie (20), dans lequel ladite unité d'endoprothèse d'introduction (2) a une longueur pouvant s'agencer avec la partie supérieure (2a) à l'intérieur de la vessie d'un patient et avec la portion inférieure (2b) agencée à l'intérieur de l'urètre avant le muscle sphincter urinaire externe du patient, en vue depuis la vessie et vers l'extérieur, dans lequel l'unité d'endoprothèse d'introduction (2) depuis la partie supérieure (2a) jusqu'à la portion inférieure (2b) a une longueur totale dans la plage de 30 à 70 mm ;
- une unité de remplissage de cathéter (3) avec une extrémité inférieure ouverte (3a) et une portion supérieure (3b), ladite unité de remplissage de cathéter (3) étant détachable du cathéter à demeure (1) ; ledit cathéter à demeure (1) comprenant également
- une unité d'obturation à ballonnet (4) en raccordement avec et ainsi pouvant se remplir par l'intermédiaire d'un tube de remplissage de ballonnet (5) et en dehors d'une ouverture d'entrée de fluide à ballonnet (21) agencée sur l'unité d'endoprothèse d'introduction (2), dans lequel le remplissage de l'unité d'obturation à ballonnet (4) avec un fluide stérile, de préférence de l'eau stérile, est prévu lorsque l'unité d'obturation à ballonnet (4) est agencée à l'intérieur de la vessie du patient pour permettre une fonction d'obturation de l'unité d'obturation à ballonnet (4) contre le col de vessie du patient ; et
- un fil de tirage (6) agencé fixement sur l'unité d'endoprothèse d'introduction (2) pour permettre le tirage de l'unité d'endoprothèse d'introduction (2) lorsque l'unité de remplissage de cathéter (3) est détachée du cathéter à demeure (1) ;
ledit nécessaire de diagnostic comprenant également
- un volume défini de solution saline, ou d'une solution correspondante, contenu dans une seringue raccordable à l'extrémité inférieure ouverte (3a) de l'unité de remplissage de cathéter (3) ; et
- un contenant de drainage et de diagnostic avec des marquages de volume ;
ledit nécessaire de diagnostic comprenant également une seringue de remplissage de ballonnet raccordable au tube de remplissage de ballonnet (5), de préférence raccordable à un réceptacle (51) situé sur l'extrémité du tube de remplissage de ballonnet (5) avant un agencement de valve (52), dans lequel ladite seringue de remplissage de ballonnet comprend un fluide stérile prévu pour remplir l'unité d'obturation à ballonnet (4).

2. Nécessaire de diagnostic selon la revendication 1, dans lequel le tube de remplissage de ballonnet (5) a une portion (50) avec une forme en spirale le long du tube de remplissage de ballonnet (5) à une position à l'extérieur et une distance depuis la portion inférieure (2b) de l'unité d'endoprothèse d'introduction (2), pour permettre l'ancrage à l'intérieur de l'urètre et à l'extérieur du muscle sphincter urinaire externe du patient, en vue depuis la vessie et vers l'extérieur.

3. Nécessaire de diagnostic selon la revendication 1 ou 2, dans lequel le fluide stérile est de l'eau stérile.

4. Utilisation d'un nécessaire de diagnostic selon de quelconques des revendications 1 à 3, pour réaliser un diagnostic dont l'objectif est d'évaluer si un patient est un candidat approprié pour une chirurgie/un traitement de la prostate ou non, sur la base du calcul de volume uriné / volume de solution saline, ou solution correspondante, s'étant écoulé dans la vessie après la réalisation du remplissage d'un volume de solution saline, ou de solution correspondante, dans la vessie du patient et la mesure du volume d'urine provenant de miction effectuée par le patient après la disjonction de l'unité de remplissage de cathéter (3) du cathéter à demeure (1).
